# FASCICULE DE BREVET EUROPEEN

(11) **EP 2 640 364 B1**
(45) Date de publication et mention de la délivrance du brevet: **18.01.2017**
(21) Numéro de dépôt: 11796758.8
(22) Date de dépôt: 18.11.2011
(51) Int. Cl.: A61K 9/20, A61K 9/28, A61K 31/19, A61K 9/50

(54) **COMPOSITION PHARMACEUTIQUE COMPRENANT DU SEL DE BICARBONATE, ET SON UTILISATION COMME MÉDICAMENT DANS LE TRAITEMENT ET/OU LA PRÉVENTION DES LITHIASES URINAIRES ET DES MALADIES LIÉES**
PHARMAZEUTISCHE ZUSAMMENSETZUNG MIT BICARBONATSALZ UND IHRE VERWENDUNG ALS ARZNEIMITTEL BEI DER BEHANDLUNG UND/ODER PRÄVENTION VON LITHIASIS DER HARNWEGE UND VERWANDTEN ERKRANKUNGEN
PHARMACEUTICAL COMPOSITION COMPRISING BICARBONATE SALT, AND USE THEREOF AS A MEDICAMENT IN THE TREATMENT AND/OR PREVENTION OF URINARY LITHIASIS AND RELATED DISEASES

(30) Priorité: 18.11.2010 FR 1059472
(43) Date de publication de la demande: 25.09.2013
(73) Titulaire: Advicenne, 30000 Nîmes (FR)
(72) Inventeur: ROUSSEL-MAUPETIT, Caroline, F-38330 Saint-ismier (FR); GRANIER, Luc-André, F-30490 Montfrin (FR); GUITTET, Catherine, F-13200 Arles (FR)
(74) Mandataire: Santarelli
(86) Numéro de dépôt international: PCT/FR2011/052695
(87) Numéro de publication internationale: WO 2012/066256

(56) Documents cités:
- EP-A1- 1 970 066
- WO-A1-97/02017
- WO-A2-2009/118359
- WO-A2-2009/140341
- GB-A- 2 130 087
- US-A- 4 451 454
- LINGAM MEKA ET AL: "Preparation of a Matrix Type Multiple-Unit Gastro Retentive Floating Drug Delivery System for Captopril Based on Gas Formation Technique: In Vitro Evaluation", AAPS PHARMSCITECH, vol. 9, no. 2, 1 juin 2008 (2008-06-01), pages 612-619, XP055003157, ISSN: 1530-9932, DOI: 10.1208/s12249-008-9090-4

## Description

La présente demande concerne une composition pharmaceutique solide à usage oral, sous forme de comprimés enrobés et à libération prolongée, ladite composition comprenant au moins un sel de bicarbonate, le comprimé étant constitué d'un coeur comprenant au moins un sel de bicarbonate en tant que principe actif et au moins une matrice hydrophile à libération prolongée, et d'un enrobage comportant au moins un agent d'enrobage, ladite composition permettant une libération continue in vivo sur une durée d'après un quart d'heure et jusqu'à douze heures après la prise d'une dose unique, La composition étant telle que sa dissolution se produit à pH indépendant dans un milieu de dissolution à pH dans une plage de 1,3 à 7, utilisée en particulier pour l'alcalinisation des urines et/ou le traitement et/ou la prévention des lithiases urinaires et des maladies liées, se manifestant à un pH physiologique et/ou lors d'acidose urinaire et/ou lors d'hypocarbonatémie et/ou lors d'hypocitraturie et/ou lors d'hypercalciurie et/ou lors d'hyperoxalurie.

Le pH physiologique urinaire est typiquement de l'ordre d'environ 5 à 5,5.

Les maladies liées sont généralement des maladies métaboliques pouvant induire comme symptômes secondaires des lithiases urinaires. L'exemple le plus classique est le Diabète de type 2 ou diabète gras qui induit une acidose et une hyperuricurie et qui, en conséquence induit des calculs urinaires d'acide urique qui peuvent être prévenus par une alcalinisation des urines.

Les maladies liées peuvent aussi être des maladies rénales comme il sera explicité ci-après.

La lithiase urinaire est une maladie consistant en la formation de calculs dans la voie urinaire. Un calcul urinaire est constitué principalement de substances cristallines. La cristallisation dépend étroitement de la saturation des urines en composés cristallisables comme le calcium, l'oxalate, le phosphore, le magnésium, le bicarbonate, l'acide urique, l'urate, le sodium ou la cystine. Ces divers composés éliminés dans les urines interviennent donc directement par leur concentration et leur tendance à cristalliser dans la formation des calculs. Mais cette tendance est aussi influencée par diverses substances inhibitrices ou inductrices de cristallisation. Ainsi le citrate et par extension les sels précurseurs du cycle de Krebs qui vont secondairement augmenter la citraturie ont une action inhibitrice sur la formation des calculs en limitant voire empêchant la croissance des cristaux, leur agrégation et leur nucléation *in vitro* et *in vivo.*

L'acidose urinaire et/ou l'hypocitraturie et/ou l'hypercalciurie et/ou l'hyperoxalurie sont des facteurs favorisants de lithiase urinaire. Un des traitements de certaines lithiases urinaires est l'alcalinisation des urines. En effet, la solubilité de certaines substances, tels la cystine et l'acide urique, est réduite en milieu acide. Des compositions alcalines comprenant des sels alcalins sont généralement indiquées en traitement de l'acidose urinaire. En outre, de nombreuses pathologies et situations thérapeutiques impliquant le rein ou d'autres organes peuvent induire une acidose métabolique. On peut citer par exemple les insuffisances rénales chroniques et aiguës, les suites de greffe rénale, ou bien les acidoses tubulaires rénales, certaines tubulopathies rénales dont la cystinurie, et aussi certaines maladies métaboliques, héréditaires ou non, tels que par exemple les acidoses tubulaires héréditaires distales (cystinoses), les diabètes et certaines intoxications.

L'alcalinisation doit permettre de maintenir en permanence le pH urinaire dans une fourchette donnée (6,5 à 7,0 en cas de cristaux oxalocalciques ou d'acide urique, 7,0 à 8,0 en cas de cristaux de cystine). Sans traitement, le pH physiologique des urines se situe en général dans une fourchette de 5,0 à 5,5, voire de 5,0 à 6,0. Ce pH peut atteindre une valeur minimale de l'ordre de 4,4. Il est d'autant plus difficile de maintenir le pH urinaire à une valeur haute que le pH physiologique des urines est bas.

Parmi les sels alcalins utilisés, on peut mentionner les sels de bicarbonate, et plus particulièrement le bicarbonate de sodium, qui est par exemple présent dans l'eau de Vichy, et le bicarbonate de potassium. Les compositions comprenant ces sels sont le plus souvent des préparations magistrales, à effet immédiat. Il faut cependant souligner le goût médiocre des sels de bicarbonate, rendant leur prise quotidienne en continu délicate.

Un certain nombre de maladies rénales entraînent une fuite de bicarbonate telles que les acidoses tubulaires distales, les maladies rénales avec perte rénale de bicarbonate (en particulier suite à des transplantations de rein ou de l'hypoplasie rénale), les insuffisances rénales avec acidoses et le syndrome de Fanconi. Les sels de bicarbonate sont couramment administrés pour pallier cette fuite de bicarbonate.

Un des avantages des sels de bicarbonate est que leur absorption dans l'organisme via le tractus intestinal est indépendante du pH. Un autre des avantages des sels de bicarbonate est qu'ils permettent de renforcer la quantité de citrate présent dans les urines. En effet, le bicarbonate est un produit de dégradation du citrate dans l'organisme. En cas de forte alcalose (due à une charge importante de bicarbonate), l'excrétion du citrate augmente via deux mécanismes : l'augmentation de la synthèse endogène de citrate et l'augmentation de la sécrétion du citrate dans les urines. Ainsi, le bicarbonate permet aussi de lutter contre l'hypocitraturie.

La prise de bicarbonate de sodium ou de bicarbonate de potassium, à fortes doses (30 à 40 grammes par jour) permet le plus souvent de maintenir en permanence le pH urinaire à plus de 7,6, mais occasionne des manifestations d'alcalose métabolique ou des troubles digestifs, principalement des diarrhées.

Par suite, un inconvénient à l'ingestion de sel de bicarbonate est sa faible tolérance gastrique à fortes doses.

La prise de bicarbonate de sodium ou de bicarbonate de potassium, à des doses plus faibles de 8 à 16 grammes par jour dans 2 à 3 litres d'eau bien répartis sur le nycthémère, permet d'alcaliniser les urines. Cependant, une telle répartition sur le nycthémère nécessite une prise toutes les deux heures, ce qui est très contraignant pour le patient. Le traitement est donc très souvent suivi de façon imparfaite.

Le document EP 1 970 066 et, surtout, la publication Breitkreutz et al., « Enteric-coated solid dosage forms containing sodium bicarbonate as a drug substance : an exception from the rule ? », JPP 2007, 59 :59-65 (2007), ainsi que la notice disponible sur le Compendium Suisse des Médicaments, décrivent un médicament, le Nephrotrans, sous forme de capsules molles comportant du bicarbonate de sodium à unité de dosage 500 mg par capsule, prescrit pour le traitement de l'hyperacidité métabolique du sang (acidose métabolique) et pour le traitement d'entretien visant à prévenir la réapparition d'une hyperacidité métabolique sanguine trop élevée lors d'insuffisance rénale chronique. L'utilisation thérapeutique de ce médicament est très différente de l'utilisation thérapeutique visée selon la présente invention, à savoir l'alcalinisation des urines et/ou le traitement et/ou la prévention des lithiases urinaires et des maladies liées, se manifestant à un pH physiologique et/ou lors d'acidose urinaire et/ou lors d'hypocarbonatémie et/ou lors d'hypocitraturie et/ou lors d'hypercalciurie et/ou lors d'hyperoxalurie. En outre, le médicament Nephrotrans présente la particularité d'être très résistant aux sucs gastriques. En effet, la publication Breitkreutz et al. démontre que, que ce soit à pH 1 ou à pH 4,5, la capsule de Nephrotrans ne produit aucune (ou quasiment aucune) libération de bicarbonate de sodium sur plusieurs heures. En outre, cette publication montre que le Nephrotrans libère le bicarbonate de sodium en 5 à 6 heures, car après 5 heures, il ne reste que 14-16% du bicarbonate de sodium dans la capsule.

En outre, le sel de bicarbonate est présent dans une proportion (appelée aussi « taux de charge ») d'environ 37,5% en poids, par rapport à la composition totale de Nephrotrans.

Le sel de bicarbonate selon Nephrotrans est donc libéré selon le pH du milieu de dissolution. *In vivo,* sa libération est donc retardée à l'entrée du produit dans l'intestin grêle, et limitée dans le temps. Une telle libération ne permet certainement pas de maintenir un pH urinaire alcalin en continu jusqu'à 8 à 12h.

En tout état de cause, l'alcalinisation des urines n'est pas optimale avec les compositions pharmaceutiques actuelles, et ce même malgré une observance correcte du traitement de base. En particulier, on note une forte baisse du pH urinaire pendant la nuit, la période la plus favorable à la lithogénèse.

En outre, le maintien d'un pH urinaire alcalin au long cours expose au risque de précipitation de phosphate de calcium rendant le calcul urinaire mixte, surtout en cas d'hypercalciurie associée. Enfin, les malades traités par l'eau de Vichy, qui contient 3,5 g/L de bicarbonate de sodium, peuvent être exposés sur le long terme à la fluorose surtout si il existe une insuffisance rénale.

Ainsi, il subsiste un besoin en une composition pharmaceutique qui permettrait avantageusement un passage du sel de bicarbonate tout au long du tractus intestinal de façon contrôlée et prolongée sur au moins environ 6 à 8 heures, de façon encore plus préférée sur plus d'environ 8 heures. La composition pharmaceutique selon l'invention permet donc généralement, et de façon particulièrement avantageuse, une libération du sel de bicarbonate essentiellement sur une période d'environ 6 à 12h, de préférence d'environ 8h à 12h.

La composition selon l'invention répond à cette problématique, car elle permet de conserver les avantages des sels de bicarbonate, tout en évitant les inconvénients cités ci-dessus.

La composition selon l'invention est une composition pharmaceutique solide à usage oral sous forme d'au moins un comprimé, le comprimé étant constitué d'un coeur comprenant au moins un sel de bicarbonate en tant que principe actif, de préférence en tant que seul principe actif, et au moins une matrice hydrophile à libération prolongée, et d'un enrobage comportant au moins un agent d'enrobage, ladite composition permettant une libération continue *in vivo* sur une durée d'après un quart d'heure et jusqu'à douze heures après la prise d'une dose unique, pour son utilisation comme médicament pour l'alcalinisation des urines et/ou dans le traitement et/ou la prévention des lithiases urinaires et des maladies liées, se manifestant à un pH physiologique et/ou lors d'acidose urinaire et/ou lors d'hypocarbonatémie et/ou lors d'hypocitraturie et/ou lors d'hypercalciurie et/ou lors d'hyperoxalurie.

De préférence, le comprimé selon l'invention consiste en un coeur et un enrobage.

Ainsi, le comprimé selon l'invention est enrobé. Selon la définition de la Pharmacopée Européenne (Ph. Eur.), un comprimé enrobé est un comprimé recouvert d'une ou plusieurs couches de mélange de substances diverses telles que résine naturelles ou synthétiques, gommes, gélatine, charges insolubles inactives, sucres, substances plastifiantes, polyols, cires, colorants autorisés par l'Autorité compétente et, parfois, aromatisants et substances actives. Toutefois, selon l'invention, il est exclu que l'enrobage comprenne un sel de bicarbonate.

Quand l'enrobage est constitué d'un film polymère très mince, le comprimé est dit pelliculé (cf. Ph. Eur.).

Avantageusement, l'enrobage permet à la fois de masquer le goût et de gérer la cinétique de libération du sel de bicarbonate.

La Pharmacopée Européenne (Ph. Eur.) définit, parmi les comprimés à libération modifiée, les comprimés à libération prolongée, les comprimés à libération retardée et les comprimés à libération séquentielle. Les comprimés à libération modifiée sont des comprimés, enrobés ou non, qui sont préparés avec des excipients spéciaux, ou par des procédés particuliers, ou les deux, visant à modifier, la vitesse, le lieu ou le moment de libération de la ou des substances actives.

En général, les comprimés à libération prolongée sont les comprimés permettant de libérer une substance active de façon prolongée dans le temps et selon une cinétique déterminée. Cela est de préférence effectué en réalisant un coeur de comprimé, ou un comprimé nu (i.e. sans enrobage) utilisant une matrice hydrophile à libération prolongée dans laquelle est présente la ou les substances actives. Une matrice à libération prolongée est en général un système matriciel, le plus souvent un réseau polymérique, qu'il soit hydrophile ou lipophile. La diffusion de la ou les substances actives au sein de ce réseau est généralement influencée non seulement par les propriétés physico-chimiques inhérentes à cette ou ces substances actives (telle que solubilité, poids moléculaire ...), mais également par celles caractérisant le réseau matriciel (tels que: hydrophilie, degré de polymérisation, vitesse de gélification, érosion).

Avantageusement, la matrice hydrophile à libération prolongée selon l'invention permet de gérer la cinétique de libération du sel de bicarbonate.

Le « coeur de comprimé » est, selon l'invention, toute la partie du comprimé qui n'est pas l'enrobage.

Par « composition pharmaceutique », on entend selon l'invention une composition dont les composants sont acceptables d'un point de vue pharmaceutique. En particulier, la composition est constituée de composants appropriés et acceptables pour une administration pharmaceutique orale.

Par « composant choisi parmi les éléments », on entend que le composant est un des éléments ou bien un mélange de ces éléments.

La composition pharmaceutique selon l'invention permet très avantageusement une libération continue *in vivo* après la prise d'une dose unique, i.e. une prise unique, sur une durée longue, généralement après un quart d'heure et jusqu'à douze heures, de façon prolongée. La libération commence généralement peu après cette prise unique, soit le plus souvent à partir d'un quart d'heure après cette prise, même si la libération peut commencer dès la prise. Par libération continue, on entend selon l'invention une libération qui s'effectue constamment *in vivo,* depuis la prise de la composition jusqu'à une durée d'environ douze heures. La cinétique de cette libération est généralement proche d'une cinétique d'ordre zéro. Une telle libération est qualifiée de « prolongée » car elle atteint ou dépasse une durée d'une heure.

De préférence, la composition pharmaceutique selon l'invention est telle qu'elle libère *in vivo* une majorité du sel de bicarbonate (c'est-à-dire au moins 50% dudit sel) sur une durée comprise entre huit et douze heures après une prise unique de la composition.

Cette libération prolongée observée *in vitro* reflète une libération contrôlée dans l'organisme, qui peut être vérifiée par mesure du pH urinaire des sujets traités par cette composition, usuellement à intervalles réguliers, par exemple toutes les deux heures.

Sans vouloir être liée par une quelconque hypothèse, la demanderesse pense que le mécanisme d'action est tel que, lorsque la composition est administrée par voie orale à un sujet, le principe actif se libère de façon contrôlée et prolongée : le sel de bicarbonate qui est absorbé tout au long du tractus digestif.

Avantageusement, la tolérance gastrique est améliorée par rapport aux compositions connues de l'art antérieur. En effet, la libération du principe actif ayant généralement lieu sur plus de huit heures, il n'y a pas d'intolérance au potassium ni d'alcalose lors de la prise de la dose. Il n'y a donc pas d'effets secondaires associés à une alcalose métabolique ou à des troubles digestifs, tels que les diarrhées.

La composition selon l'invention est particulièrement bien adaptée à l'alcalinisation des urines et/ou au traitement de l'acidose survenant lors de maladies métaboliques, rénales ou encore lors d'intoxications, et elle permet une action anti-acidose plus efficace que les formulations de l'art antérieur. Ainsi, il est possible en deux prises journalières de couvrir l'ensemble du nycthémère.

Selon une variante préférée, la composition selon l'invention est apte à libérer (ou dissoudre) le sel de bicarbonate *in vitro* dans un milieu de dissolution d'eau purifiée à pH 7 avec un appareil de dissolution de type 2, conforme à la Pharmacopée Européenne (Ph. Eur.) 2.9.3 « *Essai de dissolution des formes solides* », à un taux d'au plus 50% en 4 heures, au plus 75% en 6 heures, et au plus 90% en 8 heures.

De façon particulièrement préférée selon l'invention, indépendamment ou non de la variante précédente, la composition selon l'invention est apte à libérer (ou dissoudre) le sel de bicarbonate *in vitro* dans un milieu de dissolution d'eau purifiée à pH 7 avec un appareil de dissolution de type 2, conforme à la Pharmacopée Européenne (Ph. Eur.) 2.9.3 « *Essai de dissolution des formes solides* », à un taux compris dans une fourchette de 5% à 15% en une heure, à un taux compris dans une fourchette de 35% à 55% en cinq heures, et à un taux compris dans une fourchette de 70% à 90% en dix heures.

Ce pH de 7 est une mesure aisée à pratiquer en laboratoire, car c'est le pH de l'eau purifiée. La mesure se fait donc simplement par dissolution dans de l'eau purifiée.

Selon une variante préférée, la composition selon l'invention est apte à libérer (ou dissoudre) le sel de bicarbonate *in vitro* dans un milieu de dissolution de solution tamponnée à pH 1,3 avec un appareil de dissolution de type 2, conforme à la Pharmacopée Européenne (Ph. Eur.) 2.9.3 « *Essai de dissolution des formes solides* », à un taux d'au plus 50% en 4 heures, au plus 75% en 6 heures, et au plus 90% en 8 heures.

Selon l'invention, la composition selon l'invention est apte à libérer (ou dissoudre) le sel de bicarbonate *in vitro* dans un milieu de dissolution de solution tamponnée à pH 1,3 avec un appareil de dissolution de type 2, conforme à la Pharmacopée Européenne (Ph. Eur.) 2.9.3 « *Essai de dissolution des formes solides* », à un taux compris dans une fourchette de 5% à 15% en une heure, à un taux compris dans une fourchette de 35% à 55% en cinq heures, et à un taux compris dans une fourchette de 70% à 90% en dix heures.

De façon générale, la dissolution de la composition selon l'invention *in vitro* dans un milieu de dissolution donné, selon les conditions décrites plus haut, se produit à pH indépendant. Cela veut dire que, quelque soit le pH du milieu de dissolution dans une plage entre 1,3 et 7, la dissolution se produit selon la même cinétique. La demanderesse a choisi ici deux milieux de dissolution différents, chacun caractérisé par son pH, à savoir pH 1,3 et pH 7, pour définir ce profil de façon caractéristique, selon un test facilement reproductible *in vitro.*

Pour ces mesures, un gramme de composition pharmaceutique, ce qui correspondant à une unité de dosage, est placé dans un banc de dissolution de type Pharmatest modèle PTW S3C, dans lesquelles les conditions de température sont 37°C ± 0,5°C et la vitesse de rotation est de 100rpm (tours par minute). Le volume du bol de dissolution est de 1L et le milieu de dissolution utilisé est de l'eau purifiée à pH 7 ou une solution tamponnée à pH 1,3.

Le sel de bicarbonate est dosé comme il est connu de l'homme du métier. Par exemple, le bicarbonate de potassium libéré est dosé par conductimétrie, la technique analytique ayant été validée selon les recommandations ICH CPMP/ICH/381/95 - ICH Q2 (R1).

De préférence, le sel ne commence sa dissolution qu'après un quart d'heure (taux de dissolution généralement proche d'environ 0%), puis la cinétique de dissolution est quasiment d'ordre zéro.

Le sel de bicarbonate est de préférence choisi parmi le bicarbonate de potassium, le bicarbonate de sodium et le bicarbonate de magnésium, et de façon encore plus préférée le sel de bicarbonate est le bicarbonate de potassium.

La composition selon l'invention comprend le plus souvent de 40% à 80%, de préférence de 50 à 80%, par exemple de 50 à 70%, en poids de sel de bicarbonate sur la base du poids total de la composition. Le sel de bicarbonate est ainsi présent à dose physiologiquement efficace ou représentant un multiple ou un sous-multiple d'une dose efficace pour un patient type.

Ceci représente un taux de principe actif, en poids par rapport au poids total de la composition, important par rapport à ce qui est connu. Cela permet avantageusement de minimiser le volume de la composition pharmaceutique, et donc le volume de prise journalière. Par voie de conséquence, on obtient ainsi une meilleure acceptation par le patient.

Ceci est particulièrement appréciable pour les prises de composition en dose élevée et/ou pour les traitements thérapeutiques pédiatriques.

L'agent d'enrobage est généralement choisi parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane et l'oxyde de polyéthylène les cires de type cire de paraffine, cire d'abeille ou cire de Carnauba, les copolymères d'ammonium méthacrylate de type A et B tels que décrits dans la Pharmacopée Européenne, et les polyacrylates de dispersion environ 30% tels que décrits dans la Pharmacopée Européenne. L'agent d'enrobage est de façon préférée selon l'invention un polymère d'éthylcellulose.

Selon un mode de réalisation de l'invention, l'enrobage comporte, outre un agent d'enrobage tel que choisi dans la liste ci-dessus, un agent aromatisant et/ou un colorant.

L'épaisseur et l'homogénéité de l'enrobage est un des paramètres essentiels de l'invention, car il influence la diffusion du sel de bicarbonate au travers de l'enrobage et donc la cinétique de dissolution de ce sel. Le choix de la nature et de la quantité de l'agent d'enrobage utilisé est un paramètre important de l'invention.

La composition pharmaceutique selon l'invention comprend généralement de 1 % à 20%, de préférence de 1,5% à 3% en poids, par rapport au poids total de la composition, d'agent d'enrobage.

La matrice à libération prolongée est une matrice hydrophile, c'est-à-dire formée d'un matériau susceptible de se gélifier et d'absorber un milieu aqueux. De manière plus préférée, une telle matrice à libération prolongée est choisie parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, et les polyacrylates de dispersion environ 30% tels que décrits dans la Pharmacopée Européenne, et de façon encore plus préférée la matrice à libération prolongée est une hydroxypropylméthyl cellulose.

La composition pharmaceutique selon l'invention comprend généralement de 10% à 30%, de préférence de 15 à 25% en poids, par rapport au poids total de la composition, de matrice à libération prolongée.

La composition pharmaceutique selon l'invention peut comprendre en outre :
- de 5% à 20%, de préférence de 5% à 10% en poids, par rapport au poids total de la composition, d'un liant choisi parmi les celluloses microcristallines, la polyvidone, la polyvinylpyrrolidone, la copovidone, la gomme laque (shellac), la gélatine, les polyméthacrylates, les résines synthétiques, les acrylates, la maltodextrine, et les amidons, et de préférence le liant comporte au moins une cellulose microcristalline ;
- de 0,01% à 5%, de préférence de 0,01% à 3% en poids, par rapport au poids total de la composition, d'un agent d'écoulement choisi parmi l'acide stéarique, le polyéthylène glycol, le stéarate de magnésium, le stéarate de calcium, le stéarate de zinc, le talc, le dioxyde de silice, l'huile de castor hydrogénée, le glycéryl béhénate, et le glycéryl palmitostéarate, et de préférence l'agent d'écoulement est le stéarate de magnésium ; et/ou
- tout excipient pharmaceutique approprié, en une quantité classiquement utilisée dans le domaine considéré, par exemple de 0,0001% à 20% du poids total de la composition.

L'excipient pharmaceutique est généralement inerte, c'est-à-dire inactif et non toxique, et acceptable d'un point de vue pharmaceutique. Un tel excipient est le plus souvent choisi parmi les diluants, liants, désagrégeants, agents d'écoulement, lubrifiants, colorants autorisés par l'Administration compétente, dispersants, solubilisants, stabilisants, conservateurs, plastifiants et agents aromatisants. Un tel excipient peut être aussi un support par exemple choisi dans le groupe formé par les celluloses telles que l'hydroxyméthylcellulose, la carboxyméthylcellulose, les cyclodextrines, le polysorbate 80, le mannitol, la gélatine, le lactose, les huiles végétales, les huiles animales, les carbonates, les amidons et l'acacia.

De préférence, tous les comprimés ont la même composition et présentent un taux de dissolution similaire, qui est le taux de dissolution pouvant caractériser la composition pharmaceutique de l'invention.

Selon un mode de réalisation préféré de l'invention, la composition pharmaceutique se présente sous la forme de micro-comprimés.

La Pharmacopée Européenne (Ph. Eur.) définit un comprimé comme une préparation solide contenant une unité de prise d'une ou plusieurs substances actives. Les comprimés sont obtenus en agglomérant par compression un volume constant de particules, ou par un autre procédé de fabrication approprié tel que l'extrusion, le moulage ou la cryodessication (lyophilisation). Les comprimés sont destinés à la voie orale. Les comprimés se présentent généralement sous la forme d'un cylindre droit dans les faces inférieures et supérieures peuvent être plates ou convexes et les bords biseautés. La taille d'un comprimé, ou dimension moyenne, est donc généralement le diamètre de ce cylindre, ou un équivalent. Par contre si la hauteur du cylindre est importante, et supérieure au diamètre du cylindre, la taille du comprimé est la hauteur de ce cylindre.

Par "micro-comprimé", on entend selon l'invention un comprimé de taille comprise dans une fourchette de 2 à 4 mm (en général avec une précision sur la taille de ±10%). De préférence, tous les micro-comprimés ont la même composition et présentent un taux de dissolution similaire, qui est le taux de dissolution pouvant caractériser la composition pharmaceutique de l'invention. Ce taux de dissolution est couramment établi sur la base d'une unité de la préparation, soit dans le cadre de l'invention d'un gramme de micro-comprimés.

Du fait de la petite taille du micro-comprimé, un seul micro-comprimé ne sera pas suffisant pour une prise, et à chaque prise, plusieurs micro-comprimés seront administrés.

Un avantage de la forme en micro-comprimés est que la prise par le patient est facilitée, par rapport à une prise d'un seul comprimé de volume plus important. Ceci est particulièrement avantageux lorsque le patient est un enfant.

On comprend que selon l'invention, le patient pourra ingérer plusieurs micro-comprimés à chaque prise, selon la dose thérapeutique qui lui est appropriée (dose journalière divisée par le nombre de prises par jour). Ainsi une prise de médicament correspond à plusieurs micro-comprimés, c'est-à-dire à un ensemble de micro-comprimés. L'invention vise donc à couvrir également un ensemble de micro-comprimés, correspondant à une prise thérapeutique. L'homme du métier est à même d'évaluer le nombre de micro-comprimés correspondant à une dose thérapeutique, en fonction des besoins de la personne, de son âge, de son poids, en fonction de la quantité de sel de bicarbonate par micro-comprimé, ainsi que le nombre de prises par jour.

La composition selon l'invention n'est pas limitée à une présentation sous forme de micro-comprimés. La composition selon l'invention se présente plus généralement, dans le cadre de l'invention, sous la forme de comprimés. La taille de ces comprimés est généralement d'au moins 2 mm, par exemple comprise dans une fourchette de 2 à 25 mm. L'homme du métier est à même de choisir la taille de comprimé. Ces comprimés peuvent être des micro-comprimés, ou bien des comprimés de taille plus élevée, par exemple comprise dans une fourchette de 4 à 25 mm.

Les comprimés selon l'invention sont enrobés, ce qui permet un masquage du goût.

Les comprimés selon l'invention sont particulièrement bien adaptés à l'alcalinisation des urines, au traitement des lithiases urinaires et des maladies liées, se manifestant à un pH physiologique et/ou lors d'acidose urinaire et/ou lors d'hypocarbonatémie et/ou lors d'hypocitraturie et/ou lors d'hypercalciurie et/ou lors d'hyperoxalurie, du fait de leur profil de libération optimale.

La composition selon la présente invention peut être utilisée chez le mammifère, plus précisément chez l'homme, et tout particulièrement chez l'enfant.

Le procédé de fabrication de la composition pharmaceutique selon l'invention comprend généralement les trois étapes successives décrites ci-après :
La première étape est une étape de mélange du principe actif, de préférence du seul principe actif, avec les autres ingrédients constituant le coeur de la composition pharmaceutique selon l'invention. Le mélange est par exemple réalisé dans un mélangeur par gravité de type Stuart STR4, mais peut être réalisé dans tout autre type de mélangeur industriel.

La deuxième étape est une étape de fabrication des comprimés à partir du mélange issu de la première étape, généralement réalisée par une première opération de compression directe dans une presse rotative, par exemple pour la fabrication de micro-comprimés de dimension 2 mm (de type PR12) à l'aide de six supports possédant chacun une tête à six poinçons de 2mm. Cette deuxième étape comprend ensuite une seconde opération de dépoussiérage des comprimés fabriqués au cours de la première opération.

La troisième étape est une étape d'enrobage, par l'agent d'enrobage, des comprimés issus de la deuxième étape. L'agent d'enrobage est généralement appliqué sous forme de solution ou de suspension dans des conditions qui favorisent l'évaporation du solvant.

Selon un mode de réalisation de l'invention, la composition comprend de 60% à 70% de bicarbonate de potassium, de 15 à 25% d'hypromellose, de 7 à 17% de cellulose microcristalline, de 1 à 3% de glycéryl béhénate, de 0,01% à 1% de stéarate de magnésium, et de 1,5 à 3% d'éthyl cellulose, par rapport au poids total de la composition.

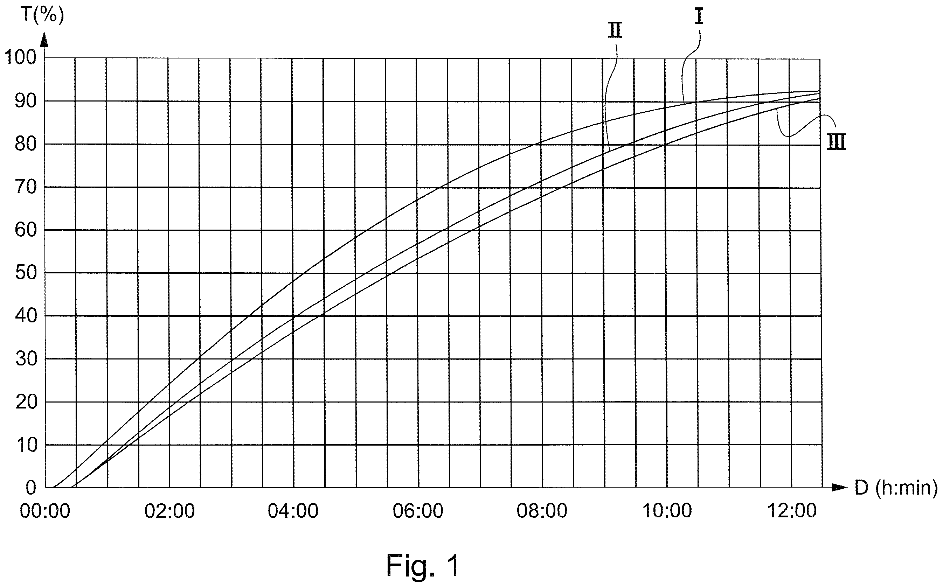

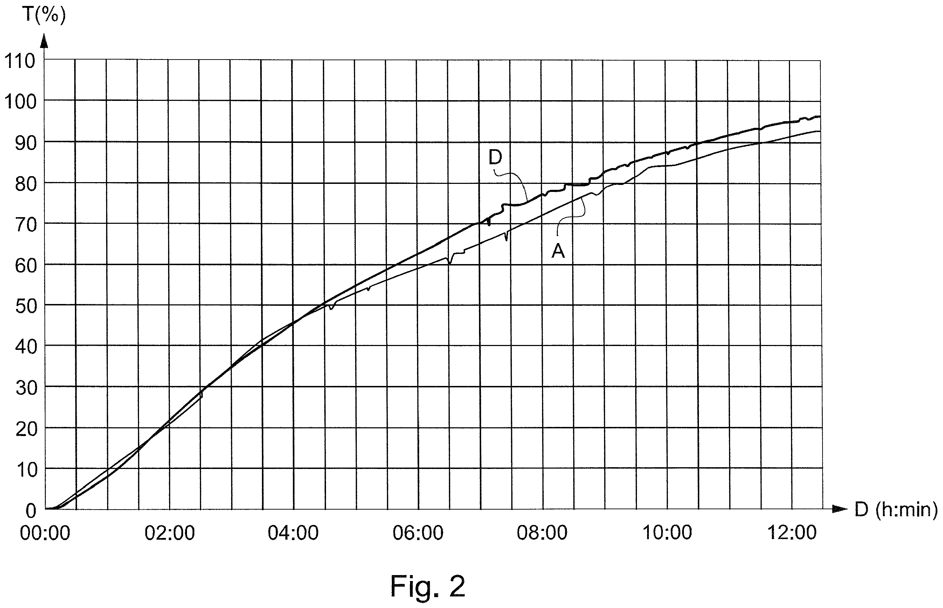

L'invention est illustrée par les figures 1 et 2 ci-jointes.

La figure 1 représente le profil de dissolution en taux T de dissolution (pourcentage de principe actif : bicarbonate de potassium) en fonction de la durée D (h:min) pour trois compositions différentes identifiées par I, Il et III.

La figure 2 représente le profil de dissolution en taux T de dissolution (pourcentage de principe actif : bicarbonate de potassium) en fonction de la durée D (h:min) pour deux compositions différentes identifiées par A et D.

La figure 1 est commentée dans l'exemple 1 ci-après.

La figure 2 est commentée dans l'exemple 2 ci-après.

Les exemples suivants illustrent l'invention sans pour autant la limiter.

### Exemple 1:

### Lot I : courbe I

Un lot I de micro-comprimés de taille (diamètre moyen) 2mm a été réalisé selon le procédé décrit précédemment, à savoir une étape de mélange des poudres, suivie d'une étape de compression puis d'une étape d'enrobage, à raison de 2000 g de micro-comprimés par lot. Ces comprimés ont la composition suivante:
Bicarbonate de potassium (principe actif, source Dr Paul Lohmann) : 66,4%
Hypromellose (matrice, HPMC 100 000 90SH0) : 19,5%
L'hypromellose est une hydroxypropylméthyl cellulose.
Cellulose microcristalline (liant, référence commerciale Ceolus® UF-711 de la société Asahi-Kasei) : 9,8%
Stéarate de magnésium (agent d'écoulement) : 0,01%
Glyceryl béhénate (agent lubrifiant, référence commerciale Compritol® ATO 888 de la société GATTEFOSSE) : 2%
Ethyl cellulose (polymère) (matériau d'enrobage, référence commerciale Ethocel® 20 standard premium de la société Dow Chemical) : 2,3%.

La courbe I de la figure 1 montre le profil de dissolution *in vitro* de tels micro-comprimés dans l'eau purifiée à pH 7.

Un tel profil a été réalisé en plaçant les mini-comprimés dans un banc de dissolution Pharmatest modèle PTW S3C, à température 37°C ± 0,5°, à volume du bol de dissolution de 1 L et à vitesse de rotation est de 100rpm.

Le dosage du bicarbonate de potassium est réalisé par conductimétrie selon une méthode analytique validée selon les recommandations ICH CPMP/ICH/381/95 - ICH Q2 (R1).

### Lot II : courbe II

Un lot II de micro-comprimés de taille (diamètre moyen) 2mm a été réalisé selon le procédé décrit précédemment, à savoir une étape de mélange des poudres, suivie d'une étape de compression puis d'une étape d'enrobage, à raison de 1 000 g de micro-comprimés par lot. Ces comprimés ont la composition suivante:
Bicarbonate de potassium (principe actif, source Dr Paul Lohmann) : 68,4%
Hypromellose (matrice, HPMC 100 000 90SH) : 19,6%
Cellulose microcristalline (liant, référence commerciale Ceolus® UF-711 de la société Asahi-Kasei) : 9,8%
Stéarate de magnésium (agent d'écoulement) : 0,06%
Ethyl cellulose (polymère) (matériau d'enrobage, référence commerciale Ethocel® 20 standard premium de la société Dow Chemical) : 2,3%.

La courbe II de la figure 1 montre le profil de dissolution *in vitro* de tels micro-comprimés dans l'eau purifiée à pH 7. Un tel profil et le dosage du bicarbonate de potassium ont été réalisés comme pour le lot I.

### Lot III : courbe III

Un lot III de micro-comprimés de taille (diamètre moyen) 2mm a été réalisé selon le procédé décrit précédemment, à savoir une étape de mélange des poudres, suivie d'une étape de compression puis d'une étape d'enrobage, à raison de 2000 g de micro-comprimés par lot. Ces comprimés ont la composition suivante:
Bicarbonate de potassium (principe actif, source Dr Paul Lohmann) : 66,4%
Hypromellose (matrice, HPMC 100 000 90SH) : 19,5%
Cellulose microcristalline (liant, référence commerciale Ceolus® UF-711 de la société Asahi-Kasei) : 9,8%
Stéarate de magnésium (agent d'écoulement) : 0,01%
Glyceryl béhénate (agent lubrifiant, référence commerciale Compritol® ATO 888 de la société GATTEFOSSE) : 2%
Ethyl cellulose (polymère) (matériau d'enrobage, référence commerciale Ethocel® 20 standard premium de la société Dow Chemical) : 2,3%.

La courbe III de la figure 1 montre le profil de dissolution *in vitro* de tels micro-comprimés dans l'eau purifiée à pH 7.

Un tel profil et le dosage du bicarbonate de potassium ont été réalisés comme pour le lot I.

Les micro-comprimés I, Il et III sont très bien acceptés et tolérés par les sujets. En outre, ils sont sans goût et faciles à avaler.

Les courbes I, Il et III ont des formes sensiblement semblables, et chacun des profils de dissolution correspondant à ces courbes I, Il et III respecte les conditions préférées revendiquées pour la composition de la présente invention.

Ainsi, chacune des trois courbes I, II et III illustre une libération de bicarbonate de potassium qui se fait de façon progressive et régulière, répondant aux critères d'un taux d'au plus 50% en 4 heures, au plus 75% en 6 heures, et au plus 90% en 8 heures.

En outre, chacune des trois courbes I, Il et III illustre une libération de bicarbonate de potassium qui aboutit à une dissolution quasiment complète au bout de 12 à 15 heures.

### Exemple 2 :

Deux lots A et D de comprimés de taille (diamètre moyen) 12 mm ont été réalisés selon le procédé décrit précédemment, à savoir une étape de mélange des poudres, suivie d'une étape de compression puis d'une étape d'enrobage, à raison de 200g de comprimés par lot. Ces comprimés ont la composition suivante :

### Courbe A : Lot A

### Bicarbonate de potassium (principe actif, source Dr Paul Lohmann) : 56%

Hypromellose (matrice à libération prolongée, HPMC 100 000 90SH, source SEPPIC) : 25%
Cire de Carnauba (matériau d'enrobage): 19%

### Courbe D : Lot D

### Bicarbonate de potassium (principe actif, source Dr Paul Lohmann) : 71%

Hypromellose (matrice à libération prolongée, HPMC 100 000 90SH, source SEPPIC) : 10%
Cire de Carnauba (matériau d'enrobage): 19%

Les courbes A et D de la figure 2 montrent les profils de dissolution *in vitro* de tels comprimés de 12 mm dans l'eau purifiée à pH 7. Les comprimés sont placés dans un banc de dissolution Pharmatest modèle PTW S3C, dans lesquelles les conditions de températures sont 37°C±0,5°, le volume du bol de dissolution est de 1 L et la vitesse de rotation est de 100rpm.

Le dosage du bicarbonate de potassium est réalisé par conductimétrie selon une méthode analytique validée selon les recommandations ICH CPMP/ICH/381/95 - ICH Q2 (R1).

Les comprimés A et D sont très bien acceptés et tolérés par les sujets. En outre, ils sont sans goût et faciles à avaler.

Les courbes A et D ont des formes sensiblement semblables, et chacun de ces profils de dissolution A et D respecte les conditions préférées revendiquées pour la composition de la présente invention.

Ainsi, chacune des deux courbes A et D illustre une libération de bicarbonate de potassium qui se fait de façon progressive et régulière, répondant aux critères d'un taux d'au plus 50% en 4 heures, au plus 75% en 6 heures, et au plus 90% en 8 heures.

En outre, chacune des deux courbes D et A illustre une libération de bicarbonate de potassium qui aboutit à une dissolution quasiment complète au bout de 12 à 15 heures.

## Revendications

1. Composition pharmaceutique solide à usage oral sous forme d'au moins un comprimé, le comprimé étant constitué d'un coeur comprenant au moins un sel de bicarbonate en tant que principe actif et au moins une matrice hydrophile à libération prolongée, et d'un enrobage comportant au moins un agent d'enrobage, ladite composition permettant une libération continue *in vivo* sur une durée d'après un quart d'heure et jusqu'à douze heures après la prise d'une dose unique,
La composition étant telle que sa dissolution se produit à pH indépendant dans un milieu de dissolution à pH dans une plage de 1,3 à 7,
pour son utilisation comme médicament pour l'alcalinisation des urines et/ou dans le traitement et/ou la prévention des lithiases urinaires et des maladies liées, se manifestant à un pH physiologique et/ou lors d'acidose urinaire et/ou lors d'hypocarbonatémie et/ou lors d'hypocitraturie et/ou lors d'hypercalciurie et/ou lors d'hyperoxalurie,
ladite composition étant apte à libérer le sel de bicarbonate *in vitro* dans un milieu de dissolution de solution tamponnée à pH 1,3 avec un appareil de dissolution de type 2, conforme à la Pharmacopée Européenne 2.9.3 « *Essai de dissolution des formes solides* », à un taux compris dans une fourchette de 5% à 15% en une heure, à un taux compris dans une fourchette de 35% à 55% en cinq heures, et à un taux compris dans une fourchette de 70% à 90% en dix heures.

2. Composition selon la revendication 1, telle qu'elle est apte à libérer le sel de bicarbonate *in vitro* dans un milieu de dissolution d'eau purifiée à pH 7 avec un appareil de dissolution de type 2, conforme à la Pharmacopée Européenne 2.9.3 « *Essai de dissolution des formes solides* », à un taux d'au plus 50% en 4 heures, au plus 75% en 6 heures, et au plus 90% en 8 heures.

3. Composition selon l'une des revendications 1 et 2, telle qu'elle est apte à libérer le sel de bicarbonate *in vitro* dans un milieu de dissolution d'eau purifiée à pH 7 avec un appareil de dissolution de type 2, conforme à la Pharmacopée Européenne 2.9.3 « *Essai de dissolution des formes solides* », à un taux compris dans une fourchette de 5% à 15% en une heure, à un taux compris dans une fourchette de 35% à 55% en cinq heures, et à un taux compris dans une fourchette de 70% à 90% en dix heures.

4. Composition selon l'une quelconque des revendications 1 à 3, telle que le sel de bicarbonate est choisi parmi le bicarbonate de potassium, le bicarbonate de sodium et le bicarbonate de magnésium, et de préférence le sel de bicarbonate est le bicarbonate de potassium.

5. Composition selon l'une quelconque des revendications 1 à 4, telle qu'elle comprend de 40% à 80%, de préférence de 50 à 80%, en poids de sel de bicarbonate sur la base du poids total de la composition.

6. Composition selon l'une quelconque des revendications 1 à 5, telle que la matrice à libération prolongée est choisie parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, et les polyacrylates de dispersion environ 30% tels que décrits dans la Pharmacopée Européenne, et de préférence la matrice à libération prolongée est une hydroxypropylméthyl cellulose.

7. Composition selon l'une quelconque des revendications 1 à 6, telle qu'elle comprend de 10% à 30%, de préférence de 15 à 25%, en poids, par rapport au poids total de la composition, de matrice à libération prolongée.

8. Composition selon l'une quelconque des revendications 1 à 7, telle que l'agent d'enrobage est choisi parmi les alginates, les polymères carboxyvinyl, les sels de sodium de carboxymethyl cellulose, les dérivés cellulosiques dont les polymères hydroxy propyl méthyl cellulose, hydroxy propyl éthyl cellulose, hydroxy propyl cellulose, hydroxy éthyl cellulose, méthyl cellulose, éthyl cellulose, la gomme de xanthane, l'oxyde de polyéthylène, les cires de type cire de paraffine, cire d'abeille ou cire de Carnauba, les copolymères d'ammonium méthacrylate de type A et B tels que décrits dans la Pharmacopée Européenne, et les polyacrylates de dispersion environ 30% tels que décrits dans la Pharmacopée Européenne, et de préférence l'agent d'enrobage est un polymère d'éthylcellulose.

9. Composition selon l'une quelconque des revendications 1 à 8, telle qu'elle comprend de 1% à 20%, de préférence de 1,5% à 3% en poids, par rapport au poids total de la composition, d'agent d'enrobage.

10. Composition selon l'une quelconque des revendications 1 à 9, telle qu'elle comprend en outre de 5% à 20%, de préférence de 5% à 10% en poids, par rapport au poids total de la composition, d'un liant choisi parmi les celluloses microcristallines, la polyvidone, la polyvinylpyrrolidone, la copovidone, la gomme laque, la gélatine, les polyméthacrylates, les résines synthétiques, les acrylates, la maltodextrine, et les amidons, et de préférence le liant comporte au moins une cellulose microcristalline.

11. Composition selon l'une quelconque des revendications 1 à 10, telle qu'elle comprend en outre de 0,01% à 5%, de préférence de 0,01% à 3% en poids, par rapport au poids total de la composition, d'un agent d'écoulement choisi parmi l'acide stéarique, le polyéthylène glycol, le stéarate de magnésium, le stéarate de calcium, le stéarate de zinc, le talc, le dioxyde de silice, l'huile de castor hydrogénée, le glycéryl béhénate, et le glycéryl palmitostéarate, et de préférence l'agent d'écoulement est le stéarate de magnésium.

12. Composition selon l'une quelconque des revendications 1 à 11, telle qu'elle se présente sous la forme de comprimés de taille comprise dans une fourchette de 2 à 25 mm.

13. Composition selon l'une quelconque des revendications 1 à 12, telle qu'elle comprend de 60% à 70% de bicarbonate de potassium, de 15 à 25% d'hypromellose, de 7 à 17% de cellulose microcristalline, de 1 à 3% de glycéryl béhénate, de 0,01 à 1% de stéarate de magnésium, et de 1,5 à 3% d'éthyl cellulose, par rapport au poids total de la composition.

## Patentansprüche

1. Feste pharmazeutische Zusammensetzung zur oralen Verwendung in Form mindestens einer Tablette, wobei die Tablette aus einem Kern mit mindestens einem Bicarbonatsalz als aktivem Wirkstoff und mindestens einer hydrophilen Retardmatrix und aus einer Beschichtung mit mindestens einem Beschichtungsmittel besteht, wobei die Zusammensetzung eine kontinuierliche Freisetzung *in vivo* über einen Zeitraum von nach einer Viertelstunde und bis zwölf Stunden nach der Einnahme einer Einzeldosis ermöglicht,
wobei die Zusammensetzung derart ist, dass ihre Auflösung in einem Lösungsmedium mit einem pH-Wert von 1,3 bis 7 unabhängig vom pH-Wert stattfindet,
für ihre Verwendung als Arzneimittel zur Harn-Alkalisierung und/oder bei der Behandlung und/oder der Vorbeugung von Urolithiasen und damit verbundener Krankheiten, die bei einem physiologischen pH-Wert und/oder bei einer Azidose der Harnwege und/oder bei einer Hypocarbonatämie und/oder bei einer Hypocitraturie und/oder bei einer Hypercalciurie und/oder bei einer Hyperoxalurie auftreten,
wobei die Zusammensetzung das Bicarbonatsalz in einem Lösungsmedium aus einer gepufferten Lösung mit einem pH-Wert von 1,3 mittels einer Lösungsvorrichtung vom Typ 2 gemäß der Europäischen Pharmakopöe 2.9.3. "*Test zur Auflösung fester Formen"* mit einer Rate in einem Bereich von 5 % bis 15 % innerhalb von einer Stunde, mit einer Rate in einem Bereich von 35 % bis 55 % innerhalb von fünf Stunden und mit einer Rate in einem Bereich von 70 % bis 90 % innerhalb von zehn Stunden *in vitro* freisetzen kann.

2. Zusammensetzung nach Anspruch 1, wobei sie das Bicarbonatsalz in einem Lösungsmedium aus gereinigtem Wasser mit einem pH-Wert von 7 mittels einer Lösungsvorrichtung vom Typ 2 gemäß der Europäischen Pharmakopöe 2.9.3. "*Test zur Auflösung fester Formen"* mit einer Rate von höchstens 50 % innerhalb von 4 Stunden, von höchstens 75 % innerhalb von 6 Stunden und von höchstens 90 % innerhalb von 8 Stunden *in vitro* freisetzen kann.

3. Zusammensetzung nach einem der Ansprüche 1 und 2, wobei sie das Bicarbonatsalz in einem Lösungsmedium aus gereinigtem Wasser mit einem pH-Wert von 7 mittels einer Lösungsvorrichtung vom Typ 2 gemäß der Europäischen Pharmakopöe 2.9.3. *"Test zur Auflösung fester Formen"* mit einer Rate in einem Bereich von 5% bis 15% innerhalb von einer Stunde, mit einer Rate in einem Bereich von 35 % bis 55 % innerhalb von fünf Stunden und mit einer Rate in einem Bereich von 70 % bis 90 % innerhalb von zehn Stunden *in vitro* freisetzen kann.

4. Zusammensetzung nach einem der Ansprüche 1 bis 3, wobei das Bicarbonatsalz aus Kaliumbicarbonat, Natriumbicarbonat und Magnesiumbicarbonat ausgewählt ist und es sich bei dem Bicarbonatsalz vorzugsweise um Kaliumbicarbonat handelt.

5. Zusammensetzung nach einem der Ansprüche 1 bis 4, wobei sie auf der Basis des Gesamtgewichts der Zusammensetzung 40 Gew.-% bis 80 Gew.-%, vorzugsweise 50 Gew.-% bis 80 Gew.-% Bicarbonatsalz umfasst.

6. Zusammensetzung nach einem der Ansprüche 1 bis 5, wobei die Retardmatrix aus den Alginaten, den Carboxyvinylpolymeren, den Salzen aus Natrium-Carboxymethylcellulose, den Cellulosederivaten einschließlich der Polymere Hydroxypropylmethylcellulose, Hydroxypropylethylcellulose, Hydroxypropylcellulose, Hydroxyethylcellulose, Methylcellulose und den Polyacrylat-Dispersionen etwa 30%, wie in der Europäischen Pharmakopöe beschrieben, ausgewählt ist und es sich bei der Retardmatrix vorzugsweise um eine Hydroxypropylmethylcellulose handelt.

7. Zusammensetzung nach einem der Ansprüche 1 bis 6, wobei sie bezogen auf das Gesamtgewicht der Zusammensetzung 10 Gew.-% bis 30 Gew.-%, vorzugsweise 15 Gew.-% bis 25 Gew.-% einer Retardmatrix umfasst.

8. Zusammensetzung nach einem der Ansprüche 1 bis 7, wobei das Beschichtungsmittel aus den Alginaten, den Carboxyvinylpolymeren, den Salzen aus Natrium-Carboxymethylcellulose, den Cellulosederivaten einschließlich der Polymere Hydroxypropylmethylcellulose, Hydroxypropylethylcellulose, Hydroxypropylcellulose, Hydroxyethylcellulose, Methylcellulose, Ethylcellulose, Xanthangummi, Polyethlylenoxid, den Wachsen vom Typ Paraffinwachs, Bienenwachs oder Carnaubawachs, den Ammoniummethacrylat-Copolymeren vom Typ A und B, wie in der Europäischen Pharmakopöe beschrieben, und den Polyacrylat-Dispersionen etwa 30%, wie in der Europäischen Pharmakopöe beschrieben, ausgewählt ist und es sich bei dem Beschichtungsmittel vorzugsweise um ein Ethylcellulosepolymer handelt.

9. Zusammensetzung nach einem der Ansprüche 1 bis 8, wobei sie bezogen auf das Gesamtgewicht der Zusammensetzung 1 Gew.-% bis 20 Gew.-%, vorzugsweise 1,5 Gew.-% bis 3 Gew.-% eines Beschichtungsmittels umfasst.

10. Zusammensetzung nach einem der Ansprüche 1 bis 9, wobei sie bezogen auf das Gesamtgewicht der Zusammensetzung ferner 5 Gew.-% bis 20 Gew.-%, vorzugsweise 5 Gew.-% bis 10 Gew.-% eines Bindemittels umfasst, das aus den mikrokristallinen Cellulosen, Polyvidon, Polyvinylpyrrolidon, Copovidon, Schellack, Gelatine, den Polymethacrylaten, den Kunstharzen, den Acrylaten, Maltodextrin und den Stärken ausgewählt ist und das Bindemittel vorzugsweise zumindest eine mikrokristalline Cellulose enthält.

11. Zusammensetzung nach einem der Ansprüche 1 bis 10, wobei sie bezogen auf das Gesamtgewicht der Zusammensetzung ferner 0,01 Gew.-% bis 5 Gew.-%, vorzugsweise 0,01 Gew.-% bis 3 Gew.-% eines Fließmittels umfasst, das aus Stearinsäure, Polyethylenglycol, Magnesiumstearat, Calciumstearat, Zinkstearat, Talk, Siliciumdioxid, hydriertem Rizinusöl, Glycerylbehenat und Glycerylpalmitostearat ausgewählt ist und es sich bei dem Fließmittel vorzugsweise um Magnesiumstearat handelt.

12. Zusammensetzung nach einem der Ansprüche 1 bis 11, wobei sie in Form von Tabletten mit einer Größe in einem Bereich von 2 bis 25 mm vorliegt.

13. Zusammensetzung nach einem der Ansprüche 1 bis 12, wobei sie bezogen auf das Gesamtgewicht der Zusammensetzung 60 % bis 70 % Kaliumbicarbonat, 15 bis 25% Hypromellose, 7% bis 17% mikrokristalline Cellulose, 1 % bis 3 % Glycerylbehenat, 0,01 % bis 1 % Magnesiumstearat und 1,5 % bis 3 % Ethylcellulose umfasst.

## Claims

1. Solid pharmaceutical composition for oral use in the form of at least one tablet, said tablet consisting of a core comprising at least one bicarbonate salt as active ingredient and at least one hydrophilic sustained-release matrix, and a coating comprising at least one coating agent, said composition permitting continuous release *in vivo* over a period of after a quarter of an hour and up to twelve hours after the administration of a single dose,
the composition being such that its dissolution is independent of the pH in a dissolution medium at a pH within a range from 1.3 to 7,
for use as a medicament for alkalization of the urine and/or in the treatment and/or prevention of urinary lithiases and related diseases, occurring at a physiological pH and/or during urinary acidosis and/or during hypobicarbonataemia and/or during hypocitraturia and/or during hypercalciuria and/or during hyperoxaluria,
said composition being able to release the bicarbonate salt *in vitro* in a dissolution medium of solution buffered at pH 1.3 with a dissolution apparatus of type 2, according to the European Pharmacopoeia 2.9.3 *"Dissolution test for solid dosage forms",* at a rate comprised within a range from 5% to 15% in one hour, at a rate comprised within a range from 35% to 55% in five hours, and at a rate comprised within a range from 70% to 90% in ten hours.

2. Composition according to the preceding claim, such that it is able to release the bicarbonate salt *in vitro* in a dissolution medium of purified water at pH 7 with a dissolution apparatus of type 2, according to the European Pharmacopoeia 2.9.3 *"Dissolution test for solid dosage forms",* at a rate of at most 50% in 4 hours, at most 75% in 6 hours, and at most 90% in 8 hours.

3. Composition according to one of claims 1 and 2, such that it is able to release the bicarbonate salt *in vitro* in a dissolution medium of purified water at pH 7 with a dissolution apparatus of type 2, according to the European Pharmacopoeia 2.9.3 *"Dissolution test for solid dosage forms",* at a rate comprised within a range from 5% to 15% in one hour, at a rate comprised within a range from 35% to 55% in five hours, and at a rate comprised within a range from 70% to 90% in ten hours.

4. Composition according to any one of claims 1 to 3, such that the bicarbonate salt is selected from potassium bicarbonate, sodium bicarbonate and magnesium bicarbonate, and preferably the bicarbonate salt is potassium bicarbonate.

5. Composition according to any one of claims 1 to 4, such that it comprises from 40% to 80%, preferably from 50 to 80%, by weight, bicarbonate salt based on the total weight of the composition.

6. Composition according to any one of claims 1 to 5, such that the sustained-release matrix is selected from alginates, carboxyvinyl polymers, sodium salts of carboxymethyl cellulose, cellulose derivatives including the polymers hydroxypropyl methylcellulose, hydroxypropyl ethylcellulose, hydroxypropyl cellulose, hydroxyethyl cellulose, methyl cellulose, and polyacrylates with dispersion of about 30% as described in the European Pharmacopoeia, and preferably the sustained-release matrix is a hydroxypropyl methylcellulose.

7. Composition according to any one of claims 1 to 6, such that it comprises from 10% to 30%, preferably from 15 to 25%, by weight, sustained-release matrix relative to the total weight of the composition.

8. Composition according to any one of claims 1 to 7, such that the coating agent is selected from alginates, carboxyvinyl polymers, sodium salts of carboxymethyl cellulose, cellulose derivatives including the polymers hydroxypropyl methylcellulose, hydroxypropyl ethylcellulose, hydroxypropyl cellulose, hydroxyethyl cellulose, methyl cellulose, ethyl cellulose, xanthan gum, polyethylene oxide, waxes such as paraffin wax, beeswax or carnauba wax, ammonium methacrylate copolymers of type A and B as described in the European Pharmacopoeia, and polyacrylates of about 30% dispersion as described in the European Pharmacopoeia, and the coating agent is preferably an ethylcellulose polymer.

9. Composition according to any one of claims 1 to 8, such that it comprises from 1% to 20%, preferably from 1.5% to 3%, by weight, coating agent relative to the total weight of the composition.

10. Composition according to any one of claims 1 to 9, such that it further comprises from 5% to 20%, preferably from 5 to 10% by weight, relative to the total weight of the composition, of a binder selected from microcrystalline celluloses, polyvidone, polyvinylpyrrolidone, copovidone, shellac, gelatin, polymethacrylates, synthetic resins, acrylates, maltodextrin, and starches, and preferably the binder comprises at least one microcrystalline cellulose.

11. Composition according to any one of claims 1 to 10, such that it further comprises from 0.01% to 5%, preferably from 0.01% to 3 % by weight, relative to the total weight of the composition, of a flow agent selected from stearic acid, polyethylene glycol, magnesium stearate, calcium stearate, zinc stearate, talc, silica, hydrogenated castor oil, glyceryl behenate, and glyceryl palmitostearate, and preferably the flow agent is magnesium stearate.

12. Composition according to any one of claims 1 to 11, such that it is in the form of tablets with a size comprised within a range from 2 to 25 mm.

13. Composition according to any one of claims 1 to 12, such that it comprises from 60% to 70% potassium bicarbonate, from 15 to 25% hypromellose, from 7 to 17% microcrystalline cellulose, from 1 to 3% glyceryl behenate, from 0.01 to 1% magnesium stearate, and from 1.5 to 3% ethyl cellulose, relative to the total weight of the composition.
